# EUROPEAN PATENT APPLICATION

(11) **EP 3 146 959 A1**
(43) Date of publication of application: **29.03.2017**
(21) Application number: 16152302.2
(22) Date of filing: 21.01.2016
(51) Int. Cl.: A61K 9/00, A61K 47/34

(54) **COMPOSITION AND USE IN TREATMENT OF A DETACHED RETINA**

(30) Priority: 16.02.2015 GB 201502565
(71) Applicant: PHARMPUR GmbH, 86343 Königsbrunn (DE)
(72) Inventor: GARVEY, Michael Joseph, Heswall, Wirral, Merseyside CH60 1XU (GB); DAY, Michael, Port Sunlight, Wirral, Merseyside CH62 5DF (GB)
(74) Representative: Leissler-Gerstl, Gabriele

(57) **Abstract**

The present invention relates to a composition comprising at least a first oil and a second oil, wherein the oils are miscible, wherein the first oil has a molecular weight of 100,000 or greater, and is present in an amount of at least 20% by weight, and wherein the second oil has a viscosity of 2,000 mPas or less.

## Description

The present invention relates to an oil composition and its use in the treatment of retinal detachment.

Retinal detachment is the separation of the neurosensory retina from its underlying pigment epithelium. Untreated, retinal detachment can result in permanent vision loss or blindness. Retinal detachment is caused by traction of the vitreous upon the retina. The traction can be 'dynamic', caused by eye movements and thus relative movement of the vitreous and the retina; or 'static', due to contraction of membranes on the surface of the retina. Retinal detachments are associated with myopia, pseudophakia, trauma and diabetes; it is often the common pathway leading to blindness in a host of ophthalmic eye diseases.

Where a retinal detachment is associated with retinal breaks (also referred to as perforations, holes or tears), fluid gains access from the vitreous cavity to the subretinal space. This form of retinal detachment is referred to as 'Rhegmatogenous'.

One effective method of closing retinal breaks involves the application of explants outside the eye in order to buckle the sclera (such as described in US 6,547,714).

Another method involves the use of internal tamponades. Internal tamponades are agents injected into the vitreous cavity to occlude retinal breaks. They are fluids that are immiscible with water and form an interface with it. The fluid can be gaseous such as air, sulphur hexafluoride (SF₆) or perfluoropropane (C₃F₈). These gases can be used undiluted in small volumes or mixed with air and totally fill the vitreous cavity. The liquids include perfluorocarbon liquids, semifluorinated alkanes or alkenes and silicone oil. Of these, only silicone oil can be tolerated in the eye for more than a few weeks. Prolonged use of any of the other liquids will give rise to retinal toxicity as demonstrated by inflammatory reaction or by histological changes.

Whilst these methods of treatment can be successful, they often require multiple treatments and there is always a danger of physical damage to the retina itself. Furthermore, there are also other dangers associated with these procedures such as the risk of infection, bleeding, high pressure inside the eye and the possibility of cataract development and these procedures often require a second operation to be undertaken.

Retinal detachments can also be treated by means of pneumatic retinopexy, whereby a gas bubble is injected into the vitreous space so as to help push the retinal tear back against the wall of the eye. This method can also be used in conjunction with the laser and cryo-surgical techniques if required. The gases preferred for such operations are commonly either perfluoropropane (C₃F₈) or sulphur hexafluoride (SF₆), which when mixed with sterile air have the properties of remaining in the eye for extended periods of time. RU2235527 discloses a number of other gases that may also be used in conjunction with this technique. Eventually, the gas is replaced by the eyes own natural fluid, although there have been recent concerns over the toxicology of compositions that are fluorine based.

Another method of treatment involves a vitrectomy whereby all or part of the vitreous gel is removed from the eye and replaced with a tamponade agent, such as a perfluorocarbon liquid, silicone oil or a gas (using a similar gaseous composition as described above) and the eye is allowed to fill with the body's own fluid over time. In this technique, a small incision is made in the wall of the eye and the vitreous gel is removed by means of a small cutting device. As the vitreous gel is removed, a saline solution is used to maintain the pressure by a continuous infusion. This solution is then exchanged with an air infusion following which an air and gas mixture is injected. Alternatively, perfluorocarbon liquids, semifluorinated alkanes or alkenes and more commonly silicone oil is injected as a tamponade agent. The tamponade agent is the immiscible fluid that occludes retinal breaks because of its interfacial tension and its buoyancy. The tamponade material is therefore intended to close the retinal tear and reoppose the retina on the underlying choroids.

As taught in, for example, WO 2006/122973, it is important to avoid emulsification of the oil within the eye. Emulsification causes a number of specific problems; firstly the emulsified droplets can stimulate adverse biological responses including inflammatory reactions. Secondly, blocking of fluid to the eye decreases aqueous outflow and can cause raised intraocular pressure and glaucoma. Thirdly, reduction of vision by opacification of the fluid within the eye, and lastly the emulsion cannot tamponade the retina.

It is also desirable to be able to inject a composition into the eye, and to remove it from the eye, at a reasonable rate and under reasonable conditions. Whilst highly viscous oils can be injected under increasingly high pressures through small incisions into the eye, the same cannot be said of removal. The maximum suction force that can be generated by a suction pump is one atmosphere pressure, and this often results in a very low flow and thus a long time for the extraction of oil. This often requires a compromise involving the use of a larger bore instrument and cannula which in turn requires the use of larger incisions into the eye. Such large incisions are undesirable surgically. They weaken the eye and increase the chances of collateral damage such as vitreous incarceration or entry site related tears. Hypotony can also follow once the oil has been removed as the balanced salt solution can now flow out relatively unimpeded via an enlarged incision. In all instances it is preferable to have small incisions with the minimal amount of invasive surgery.

It has generally been accepted that, if high molecular weight (or highly viscous) components are present in a composition, they should only be present in small amounts, to avoid slow injectability, the need for high pressure and/or the need for large incisions or large bores.

For example, WO 2006/122973 discloses compositions for use in the treatment of a detached retina, which contain only a small amount of a high molecular weight, viscous, additive, in a less viscous oil.

It is an object of the present invention to address one or more problems associated with the prior art procedures and in particular to provide a tamponade agent that can be effectively used in the treatment of retinal detachment. Furthermore, it is an object of the present invention to provide a tamponade agent that has good injectability whilst maintaining other desirable characteristics.

From a first aspect, the present invention provides a composition comprising at least a first oil and a second oil, wherein the oils are miscible, wherein the first oil has a molecular weight of 100,000 or greater, and is present in an amount of at least 20% by weight, and wherein the second oil has a viscosity of 2,000 mPas or less.

From a further aspect the present invention provides the above-mentioned composition for use in therapy.

From a further aspect the present invention provides the above-mentioned composition for use in the treatment of a detached retina.

From a further aspect the present invention provides a method of treatment of a detached retina in a patient in need thereof comprising the administration of an effective amount of the above-mentioned composition.

The present invention is directed to a composition which has a relatively large amount (compared to the prior art) of high molecular weight component diluted by a low viscosity component. Surprisingly the composition is very effective and exhibits a low injection time despite the fact that 20% or more by weight of a high molecular weight additive is used. The skilled person would expect the shear viscosity of the composition to be high because of the presence of the significant proportion of high molecular weight additive, and therefore would not expect the composition to be convenient to use. Surprisingly, despite this, the composition is very effective and is injectable in a short period of time.

Optionally the molecular weight of the first oil may be 200,000 or greater, or 300,000 or greater. Optionally the molecular weight of the first oil may be up to 1,000,000, or up to 600,000, or up to 500,000, or up to 450,000. Possible molecular weight ranges therefore include 100,000 - 1,000,000, 200,000 - 600,000, 200,000 - 500,000, and 300,000 - 450,000, for example.

Optionally, rather than defining the first oil in terms of its molecular weight, it may be defined in terms of its viscosity. The viscosity may be 10,000 mPas or greater, optionally 50,000 mPas or greater, optionally 100,000 mPas or greater, optionally 300,000 mPas or greater, optionally 500,000 mPas or greater, optionally 800,000 mPas or greater, optionally 1,000,000 mPas or greater. Optionally, the viscosity may be up to 25,000,000 mPas, optionally up to 10,000,000 mPas, optionally up to 5,000,000 mPas, optionally up to 1,000,000 mPas. Suitable ranges include, for example, 10,000 mPas to 25,000,000 mPas, 50,000 mPas to 10,000,000 mPas, or 100,000 mPas to 1,000,000 mPas.

Optionally the viscosity of the second oil may be 1,000 mPas or less, or 800 mPas or less, or 600 mPas or less, or 500 mPas or less, or 400 mPas or less. Optionally the viscosity of the second oil may be 1 mPas or greater, or 5 mPas or greater, or 10mPas or greater, or 30 mPas or greater, or 50 mPas or greater, or 80 mPas or greater. Suitable ranges include, for example, 1 - 2,000 mPas, or 1 - 1,000 mPas, or 10 - 800 mPas, or 30 - 500 mPas, or 80 - 400 mPas.

Optionally, rather than defining the second oil in terms of its viscosity, it may be defined in terms of its molecular weight. The molecular weight may be 30,000 or less, optionally 20,000 or less, or 15,000 or less. Optionally the molecular weight may be 200 or greater, or 500 or greater, or 1,000 or greater, or 3,000 or greater, or 5,000 or greater. Suitable ranges include, for example, 200 - 30,000, or 1,000 - 20,000, or 5,000 - 30,000.

The oils are miscible with each other and form a single phase, in contrast to oil combinations such as disclosed in for example Herbert et al, Graefe's Arch Clin Exp Opgthalmol 2004, 242, 250-254 wherein a silicone oil caps F₆H₈. Another point of distinction over Herbert et al. is that the present invention uses a first oil of higher molecular weight and higher viscosity.

Optionally one or both of the first oil and the second oil may be silicone oils. Optionally one or both of the first oil and the second oil may be a polydimethylsiloxane.

Optionally the amount of first oil by weight in the composition may be 25% by weight or more, or 30% by weight or more, or 40% by weight or more, e.g. 20%-45% or 25%-30% by weight.

The composition can be used to replace part or all of the vitreous gel in the posterior part of the eye.

In a further aspect of the present invention the composition may be present within a syringe or syringe component (e.g. a barrel). This can make the medical procedure easier and safer and can ensure sterility and reduce the risk of contamination associated with preparing a composition and loading it into a syringe. The composition may thus be preloaded and premixed in a ready-to-use syringe.

The process of preparation of the syringe may comprise: filling the mixture into a syringe; heat treatment; and packing and autoclaving the product. The heat treatment, for the purpose of sterilizing the mixture and the inside of the syringe, may optionally take place at 120 to 180°C. The autoclaving, for the purpose of sterilising the product on the outside, may optionally take place at 120 to 140°C.

A yet further aspect of the present invention provides for a kit of parts for producing a composition as described above comprising oils as defined above.

The kit of parts may further comprise a means by which to contact the oils together so as to form a mixture prior to use. The kit may therefore provide an apparatus such as a mixing vessel (or similar), so that the composition can be prepared prior to use. The kit may further comprise a means by which to measure a given amount of the oils prior to mixing and this will allow an individual to prepare a composition with bespoke properties prior to use.

The present invention will now be more particularly described in non-limiting detail with reference to the following examples:

### EXAMPLE 1

An experiment was conducted to examine the effect of adding a high molecular weight polydimethylsiloxane polymer (PS050 ex Fluorochem, England; molecular weight 423,000) to a low viscosity polydimethylsiloxane oil (Silicone Oil 100 mPas viscosity ex Dow Corning, USA). The amount of high molecular weight additive in the composition was 25.0% by weight.

The blend was prepared as follows. The low viscosity silicone oil was added to a polystyrene container (ex Bibby Sterilin) followed by the high molecular weight additive. The mixture was stirred for 5 days on a platform mixer (model Rotamax 120 ex Heidolph) and then vigorously mixed using an overhead stirrer for 66 hours. Finally, the mixture was stirred using a roller mixer (SRT2 ex Stuart Scientific). All mixing was done at ambient temperature (20-25°C).

The shear viscosity of the composition was measured at 25°C using a TA Instruments Advanced Rheometer AR1000. Injection speeds were measured through a 23 and 25 gauge surgical needle (both part of an Alcon Constellation Vision System Viscous Fluid Control Pack Ref: 8065750957) using an Accurus victrectomy machine. Injection tests were done at ambient temperature (20-25°C).

Silicone Oil 5000 mPas viscosity and Silicone Oil 1000 mPas viscosity (both ex Alamedics GmbH, Germany) were used as controls.

The results are shown below in Table 1.

**Table 1.**

| Composition | Shear Viscosity (Pas) | | | Injection Time (s) - 10 ml | | | |
|---|---|---|---|---|---|---|---|
| | 10 s-1 | 25s-1 | 100s-1 | 23 Gauge | 25 Gauge | | |
| | | | | Run 1 | Run 2 | | |
| Silicone Oil 1000 mPas | 1.091 | 1.093 | 1.096 | 18 | 18 | 35 | 34 |
| Silicone Oil 5000 mPas | 5.496 | 5.501 | 5.520 | 70 | 71 | 137 | 135 |
| | | | | | | | |
| **Silicone Oil 100 mPas plus 423,000 MW additive** | | | | | | | |
| | 13.44 | 12.62 | 9.318 | 24 | 24 | 48 | 44 |

It is evident that the addition of the high molecular weight additive has significantly reduced the injection time compared to the Silicone Oil 5000 control even though the shear viscosity of the test composition is much higher than that of the Silicone Oil 5000. The injection speed of the test composition also approaches that of the Silicone Oil 1000 mPas oil even though the shear viscosity of the test composition is much higher than 1000 mPas oil.

### EXAMPLE 2

An experiment was conducted to examine the effect of adding a high molecular weight polydimethylsiloxane polymer (PS049 ex Fluorochem, England; molecular weight 260,000) to a low viscosity polydimethylsiloxane oil (Silicone Oil 350 mPas viscosity ex ABLR GmbH&Co., Germany). The amount of high molecular weight additive in the composition was 25.0% by weight.

The blend was prepared as follows. The low viscosity silicone oil was added to a polystyrene container (ex Bibby Sterilin) followed by the high molecular weight additive. The mixture was stirred for 2 hours on a platform mixer (model Rotamax 120 ex Heidolph) and then vigorously mixed using an overhead stirrer for 48 hours. Finally, the mixture was stirred using a roller mixer (SRT2 ex Stuart Scientific). All mixing was done at ambient temperature (20-25°C).

The shear viscosity of the composition was measured at 25°C using a TA Instruments Advanced Rheometer AR1000. Injection speeds were measured through a 23 and 25 gauge surgical needle (both part of an Alcon Constellation Vision System Viscous Fluid Control Pack Ref: 8065750957) using an Accurus victrectomy machine. Injection tests were done at ambient temperature (20-25°C).

Silicone Oil 5000 mPas viscosity and Silicone Oil 1000 mPas viscosity (both ex Alamedics GmbH, Germany) were used as controls.

The results are shown below in Table 2.

**Table 2.**

| Composition | Shear Viscosity (Pas) | | | Injection Time (s) - 10 ml | | | |
|---|---|---|---|---|---|---|---|
| | 10 s-1 | 25s-1 | 100s-1 | 23 Gauge | | 25 Gauge | |
| | | | | Run 1 | | Run 2 | |
| Silicone Oil 1000 mPas | 1.091 | 1.093 | 1.096 | 18 | 18 | 35 | 34 |
| Silicone Oil 5000 mPas | 5.496 | 5.501 | 5.520 | 70 | 71 | 137 | 135 |
| **Silicone Oil 350 mPas plus 260,000 MW additive** | | | | | | | |
| | 7.945 | 7.850 | 7.125 | 29 | 31 | 59 | 61 |

As in Example 1, it is evident that the addition of the high molecular weight additive has significantly reduced the injection time compared to the Silicone Oil 5000 control even though the shear viscosity of the test composition is higher than that of the Silicone Oil 5000.

These experiments show that use of a large amount (over 20%) of a high molecular weight oil in combination with a low viscosity oil results in surprisingly effective injectability which is of considerable importance in the treatment of a detached retina.

## Claims

1. A composition comprising at least a first oil and a second oil, wherein the oils are miscible, wherein the first oil has a molecular weight of 100,000 or greater, and is present in an amount of at least 20% by weight, and wherein the second oil has a viscosity of 2,000 mPas or less.

2. A composition as claimed in claim 1 wherein the second oil has a viscosity of 1,000 mPas or less.

3. A composition comprising at least a first oil and a second oil, wherein the oils are miscible, wherein the first oil has a viscosity of 10,000 mPas or greater, and is present in an amount of at least 20% by weight, and wherein the second oil has a viscosity of 2,000 mPas or less.

4. A composition as claimed in claim 3 wherein the second oil has a viscosity of 1,000 mPas or less.

5. A composition comprising at least a first oil and a second oil, wherein the oils are miscible, wherein the first oil has a molecular weight of 100,000 or greater, and is present in an amount of at least 20% by weight, and wherein the second oil has a molecular weight of 30,000 or less.

6. A composition comprising at least a first oil and a second oil, wherein the oils are miscible, wherein the first oil has a viscosity of 10,000 mPas or greater, and is present in an amount of at least 20% by weight, and wherein the second oil has a molecular weight of 30,000 or less.

7. A composition as claimed in any preceding claim wherein the first oil and the second oil are silicone oils.

8. A composition as claimed in claim 5 wherein the first oil and the second oil are polydimethylsiloxanes.

9. A composition as claimed in any preceding claim for use as a medicament.

10. A composition as claimed in any preceding claim for use in the treatment of a detached retina.

11. A syringe or syringe component comprising a composition as claimed in any preceding claim.

12. Method of treatment of a detached retina comprising the use of a composition or syringe as claimed in any preceding claim.

13. A kit of parts comprising a first oil and a second oil as defined in any of claims 1 to 8.

14. A method of preparing a composition as claimed in any of claims 1 to 8 comprising mixing a first oil and a second oil as defined in any of claims 1 to 8, thereby forming a single phase mixture.
